# EUROPEAN PATENT SPECIFICATION

(11) **EP 1 086 117 B1**
(45) Date of publication and mention of the grant of the patent: **21.02.2007**
(21) Application number: 99928453.2
(22) Date of filing: 08.06.1999
(51) Int. Cl.: C07H 21/04, C07K 14/705, C12N 15/09, C12N 15/63, C12Q 1/68

(54) **CLONING AND IDENTIFICATION OF THE MOTILIN RECEPTOR**
KLONIERUNG UND IDENTIFIZIERUNG DES MOTILIN-REZEPTORS
CLONAGE ET IDENTIFICATION DU RECEPTEUR DE LA MOTILINE

(30) Priority: 12.06.1998 US 89098 P
(43) Date of publication of application: 28.03.2001
(73) Proprietor: Merck & Co., Inc., Rahway, New Jersey 07065 (US)
(72) Inventor: FEIGHNER, Scott, D., Rahway, NJ 07065 (US); PATCHETT, Arthur, A., Rahway, NJ 07065 (US); TAN, Carina, Rahway, NJ 07065 (US); MCKEE, Karen, Rahway, NJ 07065 (US); MACNEIL, Douglas, Rahway, NJ 07065 (US); HOWARD, Andrew, D., Rahway, NJ 07065 (US); PONG, Sheng-Shung, Rahway, NJ 07065 (US); SMITH, Roy, G., Rahway, NJ 07065 (US)
(74) Representative: Horgan, James Michael Frederic
(86) International application number: PCT/US1999/012773
(87) International publication number: WO 1999/064436

(56) References cited:
- US-A- 5 712 253
- FEIGHNER S D ET AL: "RECEPTOR FOR MOTILIN IDENTIFIED IN THE HUMAN GASTROINTESTINAL SYSTEM" SCIENCE, AMERICAN ASSOCIATION FOR THE ADVANCEMENT OF SCIENCE,, US, vol. 284, no. 5423, 25 June 1999 (1999-06-25), pages 2184-2188, XP001088406 ISSN: 0036-8075
- PALYHA O C ET AL: "Ligand activation domain of human orphan growth hormone (GH) secretagogue receptor (GHS-R) conserved from Pufferfish to humans" MOLECULAR ENDOCRINOLOGY, BALTIMORE, MD, US, vol. 14, no. 1, January 2000 (2000-01), pages 160-169, XP002242580 ISSN: 0888-8809
- McKEE K.K. et al., "Cloning and Characterization of Two Human G Protein-Coupled Receptor Genes (GPR38 and GPR39) Related to the Growth Hormone Secretagogue and Neurotensin Receptors", GENOMICS, 1997, Vol. 46, pages 426-434, XP002924196
- DATABASE GENEMBL, No. AF082210, PALYHA O.C. et al., "Orphan G Protein-Coupled Receptor from Teleost Fish Spheroides Nephelus Related to Growth Hormone Secretagogue Receptor", Sequence listing, September 1998.
- SATOH MINORU; SAKAI TAKAFUMI; SANO ISAMU; FUJIKURA KEIKO; KOYAMA HARUKO; OHSHIMA KIHACHI; ITOH ZEN; OMURA SATOSHI: "EM574, an Erythromycin Derivative, is a Potent Motilin Receptor Agonist in Human Gastric Antrum" JOURNAL OF PHARMACOLOGY AND EXPERIMENTAL THERAPEUTICS, vol. 271, no. 1, 1994, pages 574-579,

## Description

### FIELD OF THE INVENTION

The present invention is directed to assays using a human DN A sequence encoding a motilin receptor and the receptor encoded by the DNA.

### BACKGROUND OF THE INVENTION

Gastrointestinal (GI) motility is a coordinated neuromuscular process which transports nutrients through the digestive system, Impaired GI motility, can lead to irritable bowel syndrome, constipation and diabetic and post-surgical gastroporesis and is one of the largest health care burdens of industrialized nations. Motilin, a 22 amino acid prokinetic peptide is expressed throughout the gastrointestinal tract in a number of species including humans. Released from endochromafffin cells of the small intestine, motilin exerts a profound effect on gastric motility with the induction of interdigestive (phase III) antrum and duodenal contractions. The unrelated macrolide antibiotic erythromycin also possesses prokinetic properties mediated by its interaction with motilin receptors. These account for erythromycin's GI side-effects, including vomiting, nausea, diarrhea and abdominal muscular discomfort.

Motilin receptors have been detected in the GI tract and recently in the central nervous system, but their molecular structure has not been reported. Although motilin receptor characterization has been actively pursued in humans and other species since the isolation of motilin from porcine intestine in 1972, the receptor itself has not been isolated nor cloned.

Motilin is highly conserved across species and is synthesized as part of larger pre-prohormone. Mature 22 amino acid motilin is generated by removal of its secretory signal peptide and cleavage at the first C-terminally located dibasic prohormone convertase recognition site. Using radioligand binding, autoradiography and *in vitro* biossays, high affinity and low density, motilin receptors were detected in smooth muscle cells of the gastrointestinal tract of humans, cats and rabbits. Cerebellar brain receptors for motilin were also described supporting the notion that motilin may act in the central nervous system. Native motilin receptors appear to be coupled to G proteins and activate the phosphlipase C signal tranduction pathway resulting in Ca²⁺ influx through L-type channels.

The development of safe and selective motilin receptor agonists is likely to aid the treatment of disorders resulting from impaired GI motility. Thus, it would be desirable to be able to isolate, and clone the motilin receptor, and to use this in assays for agonists and antagonists.

Satoh et al, J. Pharm. Exp. Ther., 1994, 271 : 574-579, discloses the use of EM574 as a motilin receptor agonist in the human gastric antrum using contraction studies of muscle strips and isolated myocytes.

### SUMMARY OF THE INVENTION

The present invention is directed to assays using a G-protein coupled receptor (GPCR), designated as motilin receptor. Two spliced forms of the motilin receptor were identified: MTL-R1A, which encodes a functional seven-transmembrane domain form, and MTL-R1B, which encodes a truncated five-transmembrane domain form.

The assays are for identifying motilin ligands which are agonists and antagonists of a motilin receptor comprising contacting a candidate ligand with a motilin receptor and determining if binding occurred.

Thus, another aspect of this invention is a method for determining whether a ligand is capable of binding to a motilin receptor comprising:
(a) transfecting test cells with an expression vector encoding motilin receptor; having the amino acid sequence of SEQ ID NO:3
(b) exposing the test cells to the ligand;
(c) measuring the amount of binding of the ligand to the motilin receptor;
(d) comparing the amount of binding of the ligand to the motilin receptor in the test cells with the amount of binding of the ligand to control cells that have not been transfected with the motilin receptor
where if the amount of binding of the ligand to the test cells is greater than the amount of binding of the ligand to the control cells, then the substance is capable of binding to motilin receptor.

### BRIEF DESCRIPTION OF THE FIGURES

Figure 1 shows the DNA sequence of motilin receptor gene including 5' untranslated region (SEQ.ID.NO.:1). Intronic sequences are shown in lower case type.

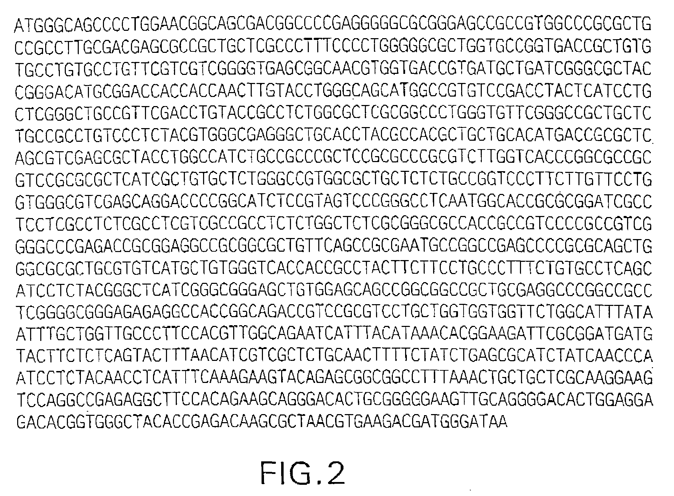

Figure 2 shows the DNA sequence of motilin receptor spliced form A (MTL-R1A) (SEQ.ID.NO.:2).

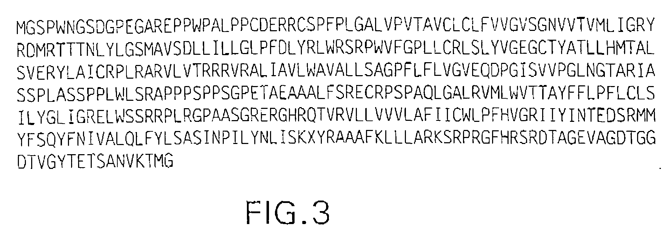

Figure 3 shows deduced amino acid sequence of MTL-R1A (SEQ.ID.NO.:3).

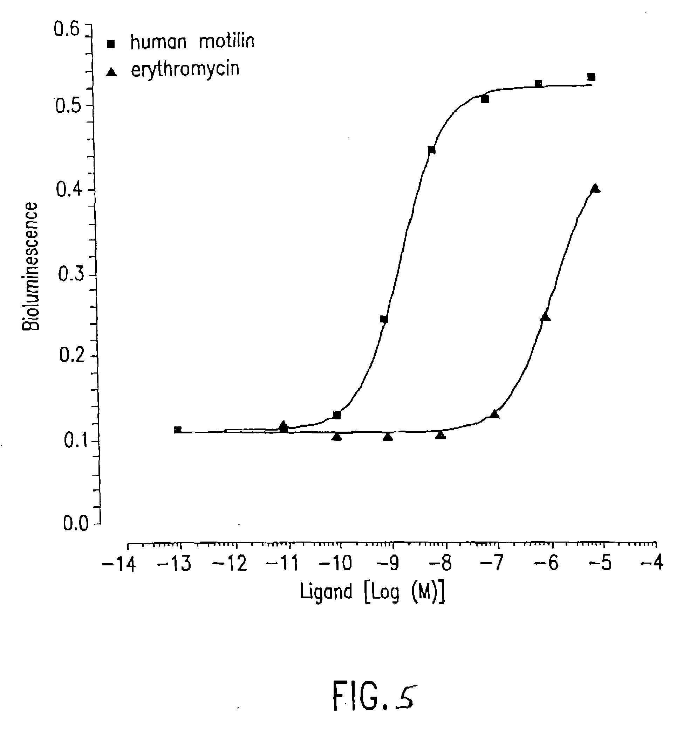

Figure 5 is a graph illustrating the pharmacological characterization of the cloned MTL-R1A in the aequorin bioluminescence assay in HEK-293 cells.

Figure 6 is a graph illustrating the pharmacological characterization of the cloned MTL-R1A in the [¹²⁵I]-Tyr⁷-human motilin binding assay.

As used throughout the specification and claims, the following definitions apply:

"Substantially free from other proteins" means at least 90%, preferably 95%, more preferably 99%, and even more preferably 99.9%, free of other proteins. Thus, for example, a MTL-R1 protein preparation that is substantially free from other proteins will contain, as a percent of its total protein, no more than 10%, preferably no more than 5%, more preferably no more than 1%, and even more preferably no more than 0.1%, of non- MTL-R1 proteins. Whether a given MTL-R1 protein preparation is substantially free from other proteins can be determined by such conventional techniques of assessing protein purity as, *e.g.,* sodium dodecyl sulfate polyacrylamide gel electrophoresis (SDS-PAGE) combined with appropriate detection methods, *e.g.,* silver staining or immunoblotting.

"Substantially free from other nucleic acids" means at least 90%, preferably 95%, more preferably 99%, and even more preferably 99.9%, free of other nucleic acids. Thus, for example, a MTL-R1 DNA preparation that is substantially free from other nucleic acids will contain, as a percent of its total nucleic acid, no more than 10%, preferably no more than 5%, more preferably no more than 1%, and even more preferably no more than 0.1%, of non- MTL-R1 nucleic acids. Whether a given MTL-R1 DNA preparation is substantially free from other nucleic acids can be determined by such conventional techniques of assessing nucleic acid purity as, *e.g.*, agarose gel electrophoresis combined with appropriate staining methods, *e.g.,* ethidium bromide staining, or by sequencing.

"Functional equivalent" means a receptor which does not have the exact same amino acid sequence of a naturally occurring motilin receptor, due to alternative splicing, deletions, mutations, or additions, but retains at least 1%, preferably 10%, and more preferably 25% of the biological activity of the naturally occurring receptor. Such derivatives will have a significant homology with a motilin receptor and can be detected by reduced stringency hybridization with a DNA sequence obtained from a motilin receptor. The nucleic acid encoding a functional equivalent has at least about 50% homology at the nucleotide level to a naturally occurring receptor nucleic acid.

"Ligand" means any molecule which binds to a motilin receptor of this invention. These ligands can have either agonist, partial agonist, partial antagonist or antagonist activity.

### DETAILED DESCRIPTION OF THE INVENTION

The cloning of GPCR's related to the hypothalamic and pituitary receptor for the growth hormone (GH) secretagogues (GHSs) which mediate sustained pulsatile GH release has been recently described. (McKee *et. al.,* 1997 *Genomics 46*:426-434, which is hereby incorporated by reference). One of these clones, GPR38, possessed the most significant amino acid sequence identity to the human GHSR (52%) (rising to as high as 86% in transmembrane domains (TM). GPR38 was classified as an orphan GPCR (GPCRs for which a natural ligand has not been identified).

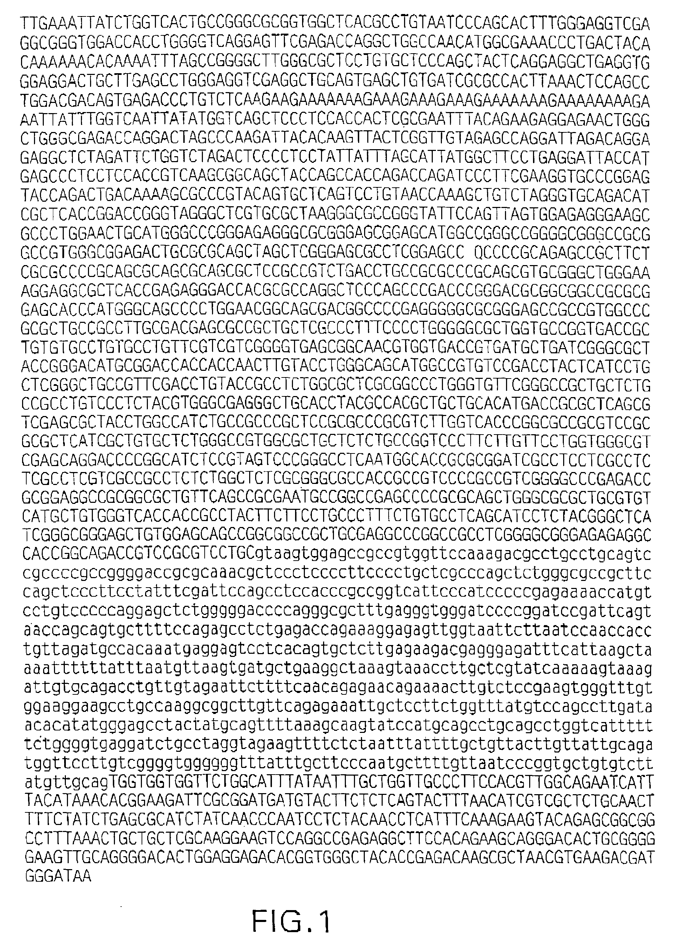

GPR38 was isolated from a human genomic DNA library and contained a single intron of approximately 1 kb, as shown in FIGURE 1. cDNA clones were isolated to obtain the nucleotide sequence of correctly spliced GPR38 mRNA. Efforts to isolate cDNA clones by standard library screening proved unsuccessful.

A combination of RACE and RT-PCR techniques resulted in the identification of two spliced forms for GPR38. These two GPR38 cDNAs use distinct splice donor sites and a common acceptor site (perfect match to consensus exon-intron splice acceptor junction sequence [pyrimidine-rich stretch ag/TG]). GPR38-A mRNA (imperfect match to consensus donor sequence [TGC/gt]) encodes a polypeptide of 412 amino acids with seven alphahelical TM domains, the hallmark feature of GPC-Rs, whereas GPR38-B encodes a 363 amino acid polypeptide with five TM domains (perfect donor sequence [CCG/gt]). Northern blot analysis failed to reveal an expression profile for GPR38. However, when RNase protection was employed expression was demonstrated in stomach, thyroid and bone marrow.

It accordance with this invention, it has been found that GPR38 is the motilin receptor.

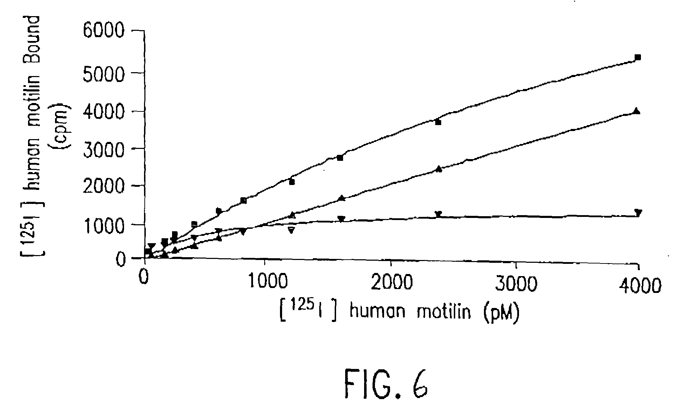

The intact motilin receptor was found to have structural features which are typical of G-protein linked receptors, including seven transmembrane (TM) domains, three intra- and extracellular loops, and the GPCR protein signature sequence. The TM domains and GPCR protein signature sequence are noted in the protein sequences of the GPCR in Figures 6A-C.

A high-throughput assay was developed which measures Ca²⁺ realease with the bioluminescent Ca²⁺ sensitive-aequorin reporter protein (capable of measuring ligand-induced IP3-coupled mobilization of intracellular calcium and concomitant calcium-induced aequorin bioluminescence). Expression of cloned GPR38-A in cell membranes was confirmed using epitope-tagged protein which revealed a single protein species with a molecular weight of approximately 45,000 daltons containing an open reading frame encoding 412 amino acids. (SEQ. ID.NO.:3). The DNA and deduced amino acid sequence are given in SEQ.ID. NO.:2 and SEQ.ID. NO.:3, respectively.

A broad set of peptide and non-peptide molecules were tested at a single concentration in transiently transfected HEK-293/aeq17 cells (100 nM peptide, 10 µM non-peptide). Significant bioluminescent responses were recorded for the peptide motilin and the non-peptide macrolide erythromycin, which was reported to be a competitive agonist at motilin receptors. Full dose-response curves confirmed this observation.

Nucleotide sequence analysis revealed two splice forms of human motilin receptor. The first (MTL-R1A) encodes a seven transmembrane domain receptor. The full length open reading frame appears to contain 412 amino acids. The second splice form (MTL-R1B) diverges in its nucleotide sequence from MTL-R1A just before the predicted amino acid of the sixth transmembrane domain (TM6).

Vectors are disclosed which comprise nucleic acids encoding a motilin receptor or a functional equivalent. These vectors may be comprised of DNA or RNA; for most cloning purposes DNA vectors are preferred. Typical vectors include plasmids, modified viruses, bacteriophage and cosmids, yeast artificial chromosomes and other forms of episomal or integrated DNA that encode a motilin receptor. It is well within the skill of the ordinary artisan to determine an appropriate vector for a particular gene transfer or other use.

Host cells are disclosed which are transformed with a gene which encodes a motilin receptor or a functional equivalent. The host cell may or may not naturally express a motilin receptor on the cell membrane. Preferrably, once transformed, the host cells are able to express the motilin receptor or a functional equivalent on the cell membrane, Depending on the host cell, it may be desirable to adapt the DNA so that particular codons are used in order to optimize expression. Such adaptations are known in the art, and these nucleic acids are also included within the scope of this invention. Generally mammalian cell lines, such as HEK-293, COS, CHO, HeLa, NS/), CV-1, GC, GH3. or VERO cells are preferred host cells, but other cells and cell lines such as *Xenopus oocytes* or insect cells, may also be used.

Human embryonic kidney (HEK 293) cells and Chinese hamster ovary (CHO) cells are particularly suitable for expression of motilin receptor proteins because these cells express a large number of G-proteins. Thus, it is likely that at least one of these G-proteins will be able to functionally couple the signal generated by interaction of motilin receptors and their ligands, thus transmitting this signal to downstream effectors, eventually resulting in a measurable change in some assayable component, *e.g.,* cAMP level, expression of a reporter gene, hydrolysis of inositol lipids, or intracellular Ca2+ levels.

A variety of mammalian expression vectors can be used to express recombinant motilin in mammalian cells. Commercially available mammalian expression vectors which are suitable include, but are not limited to, pCR2.2 (Invitrogen), pMClneo (Stratagene), pSG5 (Stratagene), pcDNAI and pcDNAIamp, pcDNA3, pcDNA3.1, pCR3.1 (Invitrogen), EBO-pSV2-neo (ATCC 37593), pBPV-1 (8-2) (ATCC 37110), pdBPV-MMTneo(342-12) (ATCC 37224), pRSVgpt (ATCC 37199), pRSVneo (ATCC 37198), and pSV2-dhfr (ATCC 37146). Following expression in recombinant cells, motilin receptors can be purified by conventional techniques to a level that is substantially free from other proteins.

The specificity of binding of compounds showing affinity for motilin receptors is shown by measuring the affinity of the compounds for recombinant cells expressing the cloned receptor or for membranes from these cells. Expression of the cloned receptor and screening for compounds that bind to motilin receptors or that inhibit the binding of a known, radiolabeled ligand of motilin receptors to these cells, or membranes prepared from these cells, provides an effective method for the rapid selection of compounds with high affinity for a motilin receptor. Such ligands need not necessarily be radiolabeled but can also be nonisotopic compounds that can be used to displace bound radiolabeled compounds or that can be used as activators in functional assays. Compounds identified by the above method are likely to be agonists or antagonists of motilin receptors and may be peptides, proteins, or non-proteinaceous organic molecules.

Such molecules are useful in treating a variety of gastric conditions, including gastric motility disorders (intrinsic myopathies or neuropathy), functional defects, disorders which are secondary to neurologic disorders including spinal cord transections, amyloidosis, collagen vascular disease (e.g. scleroderma), paraneoplastic syndromes, radiation-induced dysmotility, diabetes, infections, stress-related motiliy disorders, psychgenic/functional disorders, other drugs which affect motility (e.g. beta andrenergic drugs which may delay gastric emptying, cholinergic agents or opiates, or serotonin receptor antagonists), gastroparesis (diabetic or post-surgical), gastro-esophageal reflux disease, constipation, chronic idiopathis pseudo-obstruction and acute fecal impaction, postoperative ileus, gallstones, infantile collic, preparation for colonoscopy and endoscopy, duodenal intubation, irritable bowel syndrome, non-ulcer dyspepsion, non-cardiac chest pain and diarrhea.

The pharmacological characterization of the cloned MTL-R1A in the aequorin bioluminescence assay in HEK-293 cells is shown in Figure 10 and in the [¹²⁵I]-Tyr⁷-human motilin binding assay (Figure 11). Motilin at concentrations as high as 10 µM gave no bioluminescent response above background levels in cells that were not transfected with the MTL-R1A cDNA expression vector. Similarly, non-transfected cells did not show appreciable binding of [¹²⁵I]-Tyr⁷-human motilin.

The rank order of potency for motilin, motilin peptide fragments and non-peptide molecules is consistent with experiments performed on native motilin receptors, from stomach or intestinal tissues.

Due to the high degree of homology to GPCRs, the motilin receptor of this invention is believed to function similarly to GPCRs and have similar biological activity. They are useful in understanding the biological and physiological pathways involved in gastrointestinal motility. They may be also used to scan for motilin agonists and antagonists; as in particular to test the specificity of identified ligands.

The following, non-limiting Examples are presented to better illustrate the invention.

### EXAMPLE 1

### Sequence Comparison of MTL-R1 (GPR38) to human GHS-R, Puffer Fish 75E7 and Identification of Alternatively Spliced Forms.

Inspection of the MTL-1 genomic DNA sequence revealed two potential mRNA splice sites corresponding to consensus boundaries for exon/intron junctions. An imperfect donor site (TGC/gt) was found at nucleotides 1929-31 (Fig. 1), a perfect donor site (CCG/gt) was found at nucleotides 2080-82, and a single perfect splice acceptor site (sequence [pyrimidine-rich stretch ag/TG]) was observed at nucleotides 2729-32. To determine which splice forms exist naturally, RACE (rapid amplification of cDNA ends) was performed on thyroid poly (A)+ mRNA and RT-PCR (reverse transcriptase polymerase chain reaction) was conducted on HEK-293/aeq17 cells transfected with the MTL-1 genomic DNA construct. Directional RACE reactions were conducted on thyroid poly (A)+ mRNA that had previously been shown by RNase protection assay to contain transcripts for MTL-1R. Primer AP1 5'-CCA TCC TAA TAC GAC TCA CTA TAG GGC-3' (SEQ.ID.NO.:8) corresponds to the 5' end of the coding region including the presumptive Met initiation codon located within the cloning vector. 5'RACE1 corresponds to the 3' end of the MTL-1R coding region including the translation termination codon TAA. 5' RACE1: 5'-TTA TCC CAT CGT CTT CAC GTT AGC GCT TGT CTC-3' (SEQ.ID.NO.:9).

RACE reactions were carried out on 1 µg of thyroid poly (A)+ mRNA using the Marathon cDNA amplification/advantage PCR kit as per the manufacturer's instructions (Clontech) using the following Touchdown PCR amplification conditions: 94°C for 1 min., 5 cycles of 94°C for 30 sec. and 72°C for 4 min.; 5 cycles of 94°C for 30 sec. and 70°C for 4 min.; 25 cycles of 94°C for 20 sec and 68°C for 4 min. An approximately 1.4 kb amplified product was identified which hybridized with a ³²P-labeled probe derived from the TM 2-4 region (3F/4R probe) of the MTL-R. This product was subcloned into PCR-Script vector (Invitrogen) and sequenced.

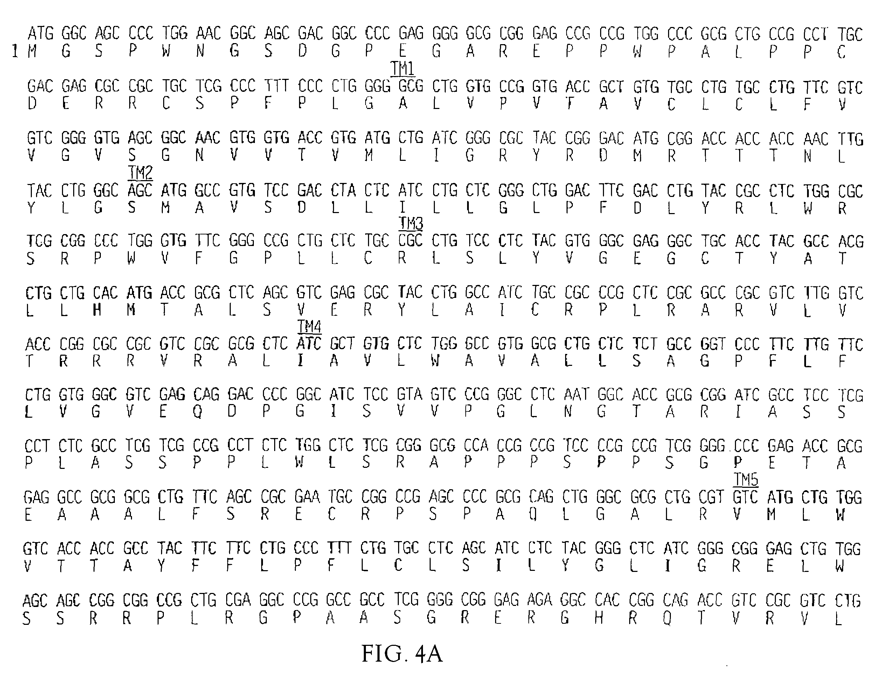

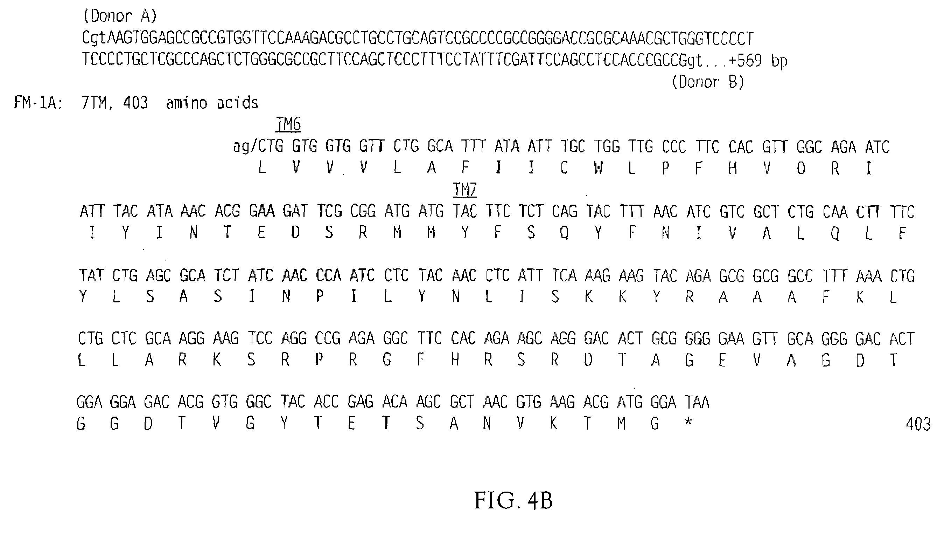

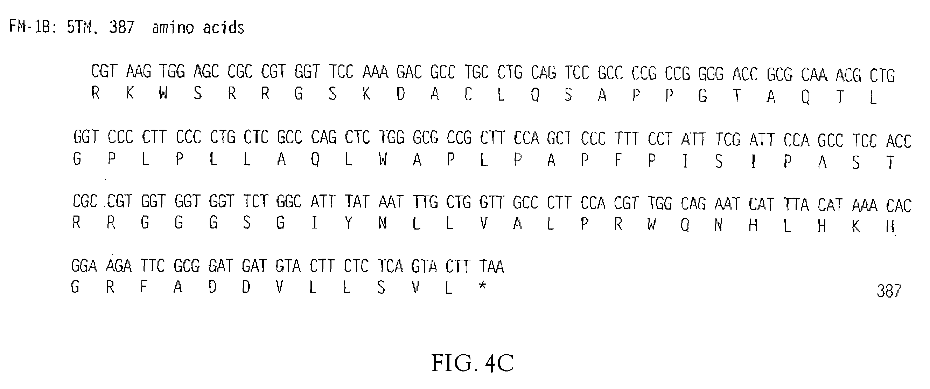

As diagrammed in Figures 4A-C, DNA sequence analysis revealed two distinct sequences corresponding to alternative use of two splice donor sequences and a common splice acceptor sequence. These results were confirmed by transfecting the MTL-1 genomic construct containing the complete ORF interrupted by a single intron of approximately 0.7 kb into HEK-293/aeq17 cells. mRNA was the isolated (Poly (A)⁺ Pure Kit, Ambion) and shown by Northern blot analysis using the 3F/4R probe to give two hybridizing bands: 2.4 kb containing the unspliced intron and approximately 1.4 kb encoding spliced forms. RT-PCR was then performed (Superscript 2 One-Step Kit, Life Technologies) on MTL-1 mRNA from transfected HEK-293/aeq17 cells using the forward primer 5' RACE1 and reverse primer 3' RACE2 (TM5 region): 5'-CTG CCC TTT CTG TGC CTC AGC ATC CTC TAC-3' (SEQ.ID.NO.:10)

An approximately 500 bp product was cloned (TA vector pCR2.2, Invitrogen), sequenced and shown to be a mixture of both splice forms. Assembly of the complete ORF for MTL-1A without intronic sequence was performed by ligation of an exon 1 fragment (Not 1 digestion of a MTL-1 plasmid containing the intron in pCDNA-3) to pCDNA-3.1 containing a Not 1/EcoR 1 exon 2 fragment.

To document protein expression, an MTL-1A plasmid encoding a amino-terminal FLAG epitope was constructed by ligation of a Pme 1 fragment from the MTL-1A/pcDNA-1.1 vector into the EcoRV site of pFLAG/CMV-2 vector (Kodak Imaging Systems). Following transfection of this plasmid into HEK-293/aeq17 cells, a protein of the expected size (approximately 48 kDa) was detected in crude cell membranes by immunoblot analysis.

### EXAMPLE 2

### Identification of Ligand Specific to Motilin Receptor

To identify a ligand for this orphan GPCR and to determine whether the full length, 7 TM domain GPR38-A is a functional GPCR, a high-throughput assay was developed which measures Ca2+ release with the bioluminescent Ca2+ sensitive aequorin reporter protein (capable of measuring ligand-induced IP₃-coupled mobilization of intracellular calcium and concomitant calcium-induced aequorin bioluminescence). Expression of GPR38-A in cell membranes was confirmed using epitope-tagged protein which revealed a single protein species with a molecular weight of approximately 45,000 daltons.

A broad set of peptide and non-peptide molecules was tested at a single concentration in transiently transfected HEK-293/aeq17 cells (100 nM peptide, 10 µM non-peptide). Significant bioluminescent responses (> 4-fold over background) were recorded for the peptide motilin and the non-peptide macrolide erythromycin, which was reported to be a competitive agonist at motilin receptors. Full dose-response curves confirmed this observation. The half-maximal effective concentration (EC₅₀) for human/porcine motilin was 2.1 +/- 0.5 nM motilin whereas erythromycin was considerably less potent (2000 +/- 210 nM; as expected from studies performed on native motilin receptors).

The signal tranduction pathway for the cloned GPR38-A motilin receptor (MTL-R1A) is through activation of phospholipase C, which has been reported for native motilin receptors. Direct radioligand binding studies with [¹²⁵I] human motilin on cell membranes prepared from transfected cells show that MTL-R1A confers high affinity and specific binding (K_{d}= 0.1 nM; Bₘₐₓ= 240 fmol/mg protein) which are strongly G protein coupled (> 80% inhibition of binding with 100 nM GTPγS).

### EXAMPLE 3

### Functional Activation of the MTL-1A Receptor

The aequorin bioluminescence assay is a reliable test for identifying G protein-coupled receptors which couple through the Gα protein subunit family consisting of G_{q} and G₁₁ which leads to the activation of phospholipase C, mobilization of intracellular calcium and activation of protein kinase C. Measurement of MTL-1A expression in the aequorin-expressing stable reporter cell line 293-AEQ 17 (Button, D. et. al.,1993 *Cell Calcium 14:* p. 663-671.) was performed using a Luminoskan RT luminometer (Labsystems Inc., Gaithersburg, MD).

293-AEQ17 cells (8 x 105 cells plated 18 hrs. before transfection in a T75 flask) were transfected with 22 µg of human MTL-R1A plasmid DNA: 264 µg lipofectamine. Following approximately 40 hours of expression the apo-aequorin in the cells was charged for 4 hours with coelenterazine (10 µM) under reducing conditions (300 µM reduced glutathione) in ECB buffer (140 mM NaCl, 20 mM KCl, 20 mM HEPES-NaOH [pH=7.4], 5 mM glucose, 1 mM MgCl₂, 1 mM CaCl₂, 0.1 mg/ml bovine serum albumin). The cells were harvested, washed once in ECB medium and resuspended to 500,000 cells/ml. 100 µl of cell suspension (corresponding to 5x104 cells) was then injected into the test plate, and the integrated light emission was recorded over 30 seconds, in 0.5 second units. 20 µL of lysis buffer (0.1% final Triton X-100 concentration) was then injected and the integrated light emission recorded over 10 seconds, in 0.5 second units. The "fractional response" values for each well were calculated by taking the ratio of the integrated response to the initial challenge to the total integrated luminescence including the Triton X-100 lysis response.

### EXAMPLE 4

### Binding of [¹²⁵I] Human Motilin to Crude Membranes from HEK-293 Cells transfected with the MTL-R1A cDNA.

The binding of [¹²⁵I] human motilin to crude membranes prepared from HEK-293/aeq17 cell transfectants was performed as follows. Crude cell membranes were prepared on ice, 48 hrs. post-transfection. Each T-75 flask was washed twice with 10 ml of PBS, once with 1 ml homogenization buffer (50 mM Tris-HCl [pH 7.4], 10 mM MgCl₂. 10 ml of homogenization buffer was added to each flask, cells were removed by scraping and then homogenized using a Polytron device (Brinkmann, Syosset, NY; 3 bursts of 10 sec. at setting 4). The homogenate was centrifuged for 20 min. at 11,000 x g at 0°C and the resulting crude membrane pellet (chiefly containing cell membranes and nuclei) was resuspended in homogenization buffer supplemented with 1.5 % BSA (0.5 ml T75 flask) and kept on ice.

Binding reactions were performed at 20°C for 1 hr. in a total volume of 0.5 ml containing: 0.1 ml of membrane suspension (approximately 1 µg protein), 10 µl of ¹²⁵I-human motilin, 10 µl of competing drug and 380-390 µl of homogenization buffer. Bound radioligand was separated by rapid vacuum filtration (Brandel 48-well cell harvester) through GF/C filters pretreated for 1 hr. with 0.5% polyethylenimine. After application of the membrane suspension to the filter, the filters were washed 3 times with 3 ml each of ice-cold 50 mM Tris-HCl [pH 7.4], 10 mM MgCl₂, and the bound radioactivity on the filters was quantitated by gamma counting. Specific binding (> 90% of total) is defined as the difference between total binding and non-specific binding conducted in the presence of 100 nM unlabeled human motilin. Competition binding data were analyzed by a nonlinear curve-fitting program (Prism V, version 2.0; GraphPad Software, San Diego, CA). Results shown are the means (+/- SEM) of triplicate determinations; Human motilin was radiolabeled with 1251 at ⁷Tyr to a specific activity of approximately 2000 Ci/mmol (Woods Assay, Portland, OR).

Structure-function analysis suggest that the motilin peptide minimally contains an N-terminal region (amino acids 1-7) essential for activity, linked to a C-terminal alpha helical domain which stabilizes the N-terminal active site region activity. The rank order of potency of several motilin peptide analogs in the MTL1-A functional and binding assays correlates with their reported potency measured by *in vitro* contractility assays (Table 1) performed on native motilin receptors in intestinal tissue. These results are summarized in Table 1 below.

| | Cloned MTL-1A Receptor (human) | |
|---|---|---|
| Ligand | Aequorin Assay (EC₅₀ nM) | [¹²⁵I] hmotilin binding (IC₅₀,nM) |
| human motilin (MTL) | 2.1 | 0.5 |
| erythromycin | 2000 | 427 |
| roxithromycin | 1950 | 613 |
| metoclopramide | > 10,000 | > 10,000 |
| cisapride | > 10,000 | > 10,000 |
| canine motilin | 4.4 | 0.2 |
| Leu 13 MTL | 3.9 | 0.2 |
| 1-11 MTL | 138 | 127 |
| 1-12 MTL | 72 | 14 |
| 1-13 MTL | 3.8 | 0.9 |
| 1-19 MTL | 4.1 | 0.3 |
| 10-22 MTL | >10,000 | 1100 |

The unrelated prokinetic agents metoclopromide and cisapride which have affinity for dopamine and/or 5-HT receptors were inactive, even at high (10 µM) doses.

### EXAMPLE 5

### Southern Blot Analysis

A genomic Southern blot (EcoRI and BamH1-digested DNA, 10 µg/lane) was hybridized with the ORF of MTL-1A. Post-hybridizational washing stringencies were at 55°C 4 X SSPE after which the filters were dried and exposed to X-ray film for 5 days at -70°C. Lambda Hind III DNA markers were (in kb), 23.1, 9.4, 6.6, 4.4, 2.3, 2.1. Southern blot analysis conducted in a variety of mammalian and non-mammalian species revealed a simple hybridization pattern consistent with a single, conserved gene encoding MTL-1A.

### EXAMPLE 7

### Expression of the MTL-R1A Gene

Transcripts of MTL-1A were detected by RNase Protection Assay (RPA). Synthesis of high-specific activity radiolabeled antisense probes and the RPA was conducted using a kit (MAXIscript and HybSpeed RPA kits; Ambion, Austin, TX) essentially as described by the manufacturer. The anti-sense cRNA MTL-1A probe was synthesized from a cDNA template encompassing nt 1234 to 1516 of the human MTL-1A inserted behind the T7 promoter in pLitmus 28 (New England Biolabs, Beverly, MA). Digestion of the construct with Stu I generated a cRNA transcript approximately 340 nt in size with approximately 60 nt of vector sequence. Input poly A⁺ mRNA (Clontech, Palo Alto, CA) was 5 g for the MTL-1A probe and 0.1 µg for a control human actin probe. Precipitated fragments were subjected to slab-gel electrophoresis (42 cm x 32 cm x 0.4 mm) in 5 % acrylamide/Tris-borate-EDTA buffer containing 8 M urea. The gels were fixed, dried and autoradiographed on film (X-Omat; Kodak, Rochester, NY) for 1-3 days (MTL-1A) or 2 hrs. (actin).

The distribution profile of MTL-1A mRNA was examined in a panel of GI and non-GI human tissues. MTL-1A mRNA could be detected in whole stomach (most prominently), thyroid, and bone marrow but was absent from several brain regions and other non-CNS tissues.

## Claims

1. A method for determining whether a ligand is capable of binding to a motilin receptor comprising:
(a) transfecting test cells with an expression vector encoding motilin receptor having the amino acid sequence of SEQ.ID.NO.:3;
(b) exposing the test cells to the ligand;
(c) measuring the amount of binding of the ligand to the motilin receptor;
(d) comparing the amount of binding of the ligand to the motilin receptor in the test cells with the amount of binding of the ligand to control cells that have not been transfected with the motilin receptor
where if the amount of binding of the ligand to the test cells is greater than the amount of binding of the ligand to the control cells, then the substance is capable of binding to motilin receptor.

2. A method according to claim 1 where the nucleic acid sequence encoding the motilin receptor is as shown in SEQ.ID.NO:2.

## Patentansprüche

1. Verfahren zur Feststellung, ob ein Ligand zur Bindung an einen Motilin-Rezeptor in der Lage ist, umfassend:
a) Transfizieren von Testzellen mit einem Expressionsvektor, der für einen Motilin-Rezeptor mit der Aminosäuresequenz von SEQ-ID-Nr. 3 codiert;
b) Aussetzen der Testzellen dem Liganden;
c) Messen des Ausmaßes der Bindung des Liganden an den Motilin-Rezeptor;
d) Vergleichen des Ausmaßes der Bindung des Liganden an den Motilin-Rezeptor in den Testzellen mit dem Ausmaß der Bindung des Liganden an Kontrollzellen, die nicht mit dem Motilin-Rezeptor transfiziert wurden,
wobei die Substanz zur Bindung an den Motilin-Rezeptor in der Lage ist, wenn das Ausmaß der Bindung des Liganden an die Testzellen größer ist als das Ausmaß der Bindung des Liganden an die Kontrollzellen.

2. Verfahren nach Anspruch 1, wobei die Nukleinsäuresequenz, die für den Motilin-Rezeptor codiert, wie in SEQ-ID-Nr. 2 dargestellt ist.

## Revendications

1. Méthode pour déterminer si un ligand est capable de se lier à un récepteur de la motiline comprenant les étapes consistant à :
(a) transfecter des cellules tests avec un vecteur d'expression codant pour le récepteur de la motiline ayant la séquence d'acides aminés de la SEQ. ID. n° : 3 ;
(b) exposer les cellules tests au ligand ;
(c) mesurer la quantité de liaison du ligand au récepteur de la motiline ;
(d) comparer la quantité de liaison du ligand au récepteur de la motiline dans les cellules tests avec la quantité de liaison du ligand à des cellules de contrôle qui n'ont pas été transfectées avec le récepteur de la motiline
dans laquelle si la quantité de liaison du ligand aux cellules tests est supérieure à la quantité de liaison du ligand aux cellules de contrôle, alors la substance est capable de se lier au récepteur de la motiline.

2. Méthode selon la revendication 1 dans laquelle la séquence d'acides nucléiques codant pour le récepteur de la motiline est telle que présentée dans la SEQ. ID. n° : 2.


### SEQUENCE LISTING

(1) GENERAL INFORMATION:
   (i) APPLICANT: Merck & Co., Inc.
   (ii) TITLE OF INVENTION: CLONING AND IDENTIFICATION OF THE MOTILIN RECEPTOR
   (iii) NUMBER OF SEQUENCES: 15
   (iv) CORRESPONDENCE ADDRESS:
      (A) ADDRESSEE: Merck & Co., Inc.
      (B) STREET: P.O. Box 2000, 126 E. Lincoln Ave.
      (C) CITY: Rahway
      (D) STATE: NJ
      (E) COUNTRY: USA
      (F) ZIP: 07065-0900
   (v) COMPUTER READABLE FORM:
      (A) MEDIUM TYPE: Diskette
      (B) COMPUTER: IBM Compatible
      (C) OPERATING SYSTEM: Windows
      (D) SOFTWARE: FastSEQ for Windows Version 2.0b
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 60/089,098
      (B) FILING DATE: 12-JUN-1998
   (viii) ATTORNEY/AGENT INFORMATION:
      (A) NAME: Giesser, Joanne M
      (B) REGISTRATION NUMBER: 32,838
      (C) REFERENCE/DOCKET NUMBER: 20251 PCT
   (ix) TELECOMMUNICATION INFORMATION:
      (A) TELEPHONE: 732-594-3046
      (B) TELEFAX: 732-594-4720
      (C) TELEX:
(2) INFORMATION FOR SEQ ID NO:1:
   (i) SEQUENCE CHARACTERISTICS:
      (A) LENGTH: 3066 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:1:
   TTGAAATTAT CTGGTCACTG CCGGGCGCGG TGGCTCACGC CTGTAATCCC AGCACTTTGG 60
   GAGGTCGAGG CGGGTGGACC ACCTGGGGTC AGGAGTTCGA GACCAGGCTG GCCAACATGG 120
   CGAAACCCTG ACTACACAAA AAACACAAAA TTTAGCCGGG GCTTGGGCGC TCCTGTGCTC 180
   CCAGCTACTC AGGAGGCTGA GGTGGGAGGA CTGCTTGAGC CTGGGAGGTC GAGGCTGCAG 240
   TGAGCTGTGA TCGCGCCACT TAAACTCCAG CCTGGACGAC AGTGAGACCC TGTCTCAAGA 300
   AGAAAAAAAG AAAGAAAGAA AGAAAAAAAG AAAAAAAAGA AATTATTTGG TCAATTATAT 360
   GGTCAGCTCC CTCCACCACT CGCGAATTTA CAGAAGAGGA GAACTGGGCT GGGCGAGACC 420
   AGGACTAGCC CAAGATTACA CAAGTTACTC GGTTGTAGAG CCAGGATTAG ACAGGAGAGG 480
   CTCTAGATTC TGGTCTAGAC TCCCCTCCTA TTATTTAGCA TTATGGCTTC CTGAGGATTA 540
   CCATGAGCCC TCCTCCACCG TCAAGCGGCA GCTACCAGCC ACCAGACCAG ATCCCTTCGA 600
   AGGTGCCCGG AGTACCAGAC TGACAAAAGC GCCCGTACAG TGCTCAGTCC TGTAACCAAA 660
   GCTGTCTAGG GTGCAGACAT CGCTCACCGG ACCGGGTAGG GCTCGTGCGC TAAGGGCGCC 720
   GGGTATTCCA GTTAGTGGAG AGGGAAGCGC CCTGGAACTG CATGGGCCCG GGAGAGGGCG 780
   CGGGAGCGGA GCATGGCCGG GCCGGGGCGG GCCGCGGCCG TGGGCGGAGA CTGCGCGCAG 840
   CTAGCTCGGG AGCGCCTCGG AGCCCACCCC GCAGAGCCGC TTCTCGCGCC CCGCAGCGCA 900
   GCGCAGCGCT CCGCCGTCTG ACCTGCCGCG CCCGCAGCGT GCGGGCTGGG AAAGGAGGCG 960
   CTCACCGAGA GGGACCACGC GCCAGGCTCC CAGCCCGACC CGGGACGCGG CGGCCGCGCG 1020
   GAGCACCCAT GGGCAGCCCC TGGAACGGCA GCGACGGCCC CGAGGGGGCG CGGGAGCCGC 1080
   CGTGGCCCGC GCTGCCGCCT TGCGACGAGC GCCGCTGCTC GCCCTTTCCC CTGGGGGCGC 1140
   TGGTGCCGGT GACCGCTGTG TGCCTGTGCC TGTTCGTCGT CGGGGTGAGC GGCAACGTGG 1200
   TGACCGTGAT GCTGATCGGG CGCTACCGGG ACATGCGGAC CACCACCAAC TTGTACCTGG 1260
   GCAGCATGGC CGTGTCCGAC CTACTCATCC TGCTCGGGCT GCCGTTCGAC CTGTACCGCC 1320
   TCTGGCGCTC GCGGCCCTGG GTGTTCGGGC CGCTGCTCTG CCGCCTGTCC CTCTACGTGG 1380
   GCGAGGGCTG CACCTACGCC ACGCTGCTGC ACATGACCGC GCTCAGCGTC GAGCGCTACC 1440
   TGGCCATCTG CCGCCCGCTC CGCGCCCGCG TCTTGGTCAC CCGGCGCCGC GTCCGCGCGC 1500
   TCATCGCTGT GCTCTGGGCC GTGGCGCTGC TCTCTGCCGG TCCCTTCTTG TTCCTGGTGG 1560
   GCGTCGAGCA GGACCCCGGC ATCTCCGTAG TCCCGGGCCT CAATGGCACC GCGCGGATCG 1620
   CCTCCTCGCC TCTCGCCTCG TCGCCGCCTC TCTGGCTCTC GCGGGCGCCA CCGCCGTCCC 1680
   CGCCGTCGGG GCCCGAGACC GCGGAGGCCG CGGCGCTGTT CAGCCGCGAA TGCCGGCCGA 1740
   GCCCCGCGCA GCTGGGCGCG CTGCGTGTCA TGCTGTGGGT CACCACCGCC TACTTCTTCC 1800
   TGCCCTTTCT GTGCCTCAGC ATCCTCTACG GGCTCATCGG GCGGGAGCTG TGGAGCAGCC 1860
   GGCGGCCGCT GCGAGGCCCG GCCGCCTCGG GGCGGGAGAG AGGCCACCGG CAGACCGTCC 1920
   GCGTCCTGCG TAAGTGGAGC CGCCGTGGTT CCAAAGACGC CTGCCTGCAG TCCGCCCCGC 1980
   CGGGGACCGC GCAAACGCTG GGTCCCCTTC CCCTGCTCGC CCAGCTCTGG GCGCCGCTTC 2040
   CAGCTCCCTC CTATTTCGAT TCCAGCCTCC ACCCGCCGGT ACTTCCCATC CCCCGAGAAA 2100
   ACCATGTCCT GTCCCCCAGG AGCTCTGGGG GACCCCAGGG CGCTTTGAGG GTGGGATCCC 2160
   CGGATCCGAT TCAGTAACCA GCAGTGCTTT TCCAGAGCCT CTGAGACCAG AAAGGAGAGT 2220
   TGGTAATTCT TAATCCAACC ACCTGTTAGA TGCCACAAAT GAGGAGTCCT CACAGTGCTC 2280
   TTGAGAAGAC GAGGGAGATT TCATTAAGCT AAAATTTTTT ATTTAATGTT AAGTGATGCT 2340
   GAAGGCTAAA GTAAACCTTG CTCGTATCAA AAAGTAAAGA TTGTGCAGAC CTGTTGTAGA 2400
   ATTCTTTTCA ACAGAGAACA GAAAACTTGT CTCCGAAGTG GGTTTGTGGA AGGAAGCCTG 2460
   CCAAGGCGGC TTGTTCAGAG AAATTGCTCC TTCTGGTTTA TGTCCAGCCT TGATAACACA 2520
   TATGGGAGCC TACTATGCAG TTTTAAAGCA AGTATCCATG CAGCCTGCAG CCTGGTCATT 2580
   TTTTCTGGGG TGAGGATCTG CCTAGGTAGA AGTTTTCTCT AATTTATTTT GCTGTTACTT 2640
   GTTATTGCAG ATGGTTCCTT GTCGGGGTGG GGGGTTTATT TGCTTCCCAA TGCTTTTGTT 2700
   AATCCCGGTG CTGTGTCTTA TGTTGCAGTG GTGGTGGTTC TGGCATTTAT AATTTGCTGG 2760
   TTGCCCTTCC ACGTTGGCAG AATCATTTAC ATAAACACGG AAGATTCGCG GATGATGTAC 2820
   TTCTCTCAGT ACTTTAACAT CGTCGCTCTG CAACTTTTCT ATCTGAGCGC ATCTATCAAC 2880
   CCAATCCTCT ACAACCTCAT TTCAAAGAAG TACAGAGCGG CGGCCTTTAA ACTGCTGCTC 2940
   GCAAGGAAGT CCAGGCCGAG AGGCTTCCAC AGAAGCAGGG ACACTGCGGG GGAAGTTGCA 3000
   GGGGACACTG GAGGAGACAC GGTGGGCTAC ACCGAGACAA GCGCTAACGT GAAGACGATG 3060
   GGATAA 3066
(2) INFORMATION FOR SEQ ID NO:2:
   (i) SEQUENCE CHARACTERISTICS:
      (A) LENGTH: 1239 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:2:
   ATGGGCAGCC CCTGGAACGG CAGCGACGGC CCCGAGGGGG CGCGGGAGCC GCCGTGGCCC    60
   GCGCTGCCGC CTTGCGACGA GCGCCGCTGC TCGCCCTTTC CCCTGGGGGC GCTGGTGCCG   120
   GTGACCGCTG TGTGCCTGTG CCTGTTCGTC GTCGGGGTGA GCGGCAACGT GGTGACCGTG   180
   ATGCTGATCG GGCGCTACCG GGACATGCGG ACCACCACCA ACTTGTACCT GGGCAGCATG   240
   GCCGTGTCCG ACCTACTCAT CCTGCTCGGG CTGCCGTTCG ACCTGTACCG CCTCTGGCGC   300
   TCGCGGCCCT GGGTGTTCGG GCCGCTGCTC TGCCGCCTGT CCCTCTACGT GGGCGAGGGC   360
   TGCACCTACG CCACGCTGCT GCACATGACC GCGCTCAGCG TCGAGCGCTA CCTGGCCATC   420
   TGCCGCCCGC TCCGCGCCCG CGTCTTGGTC ACCCGGCGCC GCGTCCGCGC GCTCATCGCT   480
   GTGCTCTGGG CCGTGGCGCT GCTCTCTGCC GGTCCCTTCT TGTTCCTGGT GGGCGTCGAG   540
   CAGGACCCCG GCATCTCCGT AGTCCCGGGC CTCAATGGCA CCGCGCGGAT CGCCTCCTCG   600
   CCTCTCGCCT CGTCGCCGCC TCTCTGGCTC TCGCGGGCGC CACCGCCGTC CCCGCCGTCG   660
   GGGCCCGAGA CCGCGGAGGC CGCGGCGCTG TTCAGCCGCG AATGCCGGCC GAGCCCCGCG   720
   CAGCTGGGCG CGCTGCGTGT CATGCTGTGG GTCACCACCG CCTACTTCTT CCTGCCCTTT   780
   CTGTGCCTCA GCATCCTCTA CGGGCTCATC GGGCGGGAGC TGTGGAGCAG CCGGCGGCCG   840
   CTGCGAGGCC CGGCCGCCTC GGGGCGGGAG AGAGGCCACC GGCAGACCGT CCGCGTCCTG   900
   CTGGTGGTGG TTCTGGCATT TATAATTTGC TGGTTGCCCT TCCACGTTGG CAGAATCATT   960
   TACATAAACA CGGAAGATTC GCGGATGATG TACTTCTCTC AGTACTTTAA CATCGTCGCT  1020
   CTGCAACTTT TCTATCTGAG CGCATCTATC AACCCAATCC TCTACAACCT CATTTCAAAG  1080
   AAGTACAGAG CGGCGGCCTT TAAACTGCTG CTCGCAAGGA AGTCCAGGCC GAGAGGCTTC  1140
   CACAGAAGCA GGGACACTGC GGGGGAAGTT GCAGGGGACA CTGGAGGAGA CACGGTGGGC  1200
   TACACCGAGA CAAGCGCTAA CGTGAAGACG ATGGGATAA                          1239
(2) INFORMATION FOR SEQ ID NO:3:
   (i) SEQUENCE CHARACTERISTICS:
      (A) LENGTH: 412 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:3:
   Met Gly Ser Pro Trp Asn Gly Ser Asp Gly Pro Glu Gly Ala Arg Glu
    1               5                  10                  15
   Pro Pro Trp Pro Ala Leu Pro Pro Cys Asp Glu Arg Arg Cys Ser Pro
               20                  25                  30
   Phe Pro Leu Gly Ala Leu Val Pro Val Thr Ala Val Cys Leu Cys Leu
           35                  40                  45
   Phe Val Val Gly Val Ser Gly Asn Val Val Thr Val Met Leu Ile Gly
       50                  55                  60
   Arg Tyr Arg Asp Met Arg Thr Thr Thr Asn Leu Tyr Leu Gly Ser Met
   65                  70                  75                  80
   Ala Val Ser Asp Leu Leu Ile Leu Leu Gly Leu Pro Phe Asp Leu Tyr
                   85                  90                  95
   Arg Leu Trp Arg Ser Arg Pro Trp Val Phe Gly Pro Leu Leu Cys Arg
               100                 105                 110
   Leu Ser Leu Tyr Val Gly Glu Gly Cys Thr Tyr Ala Thr Leu Leu His
           115                 120                 125
   Met Thr Ala Leu Ser Val Glu Arg Tyr Leu Ala Ile Cys Arg Pro Leu
       130                 135                 140
   Arg Ala Arg Val Leu Val Thr Arg Arg Arg Val Arg Ala Leu Ile Ala
   145                 150                 155                 160
   Val Leu Trp Ala Val Ala Leu Leu Ser Ala Gly Pro Phe Leu Phe Leu
                   165                 170                 175
   Val Gly Val Glu Gln Asp Pro Gly Ile Ser Val Val Pro Gly Leu Asn
               180                 185                 190
   Gly Thr Ala Arg Ile Ala Ser Ser Pro Leu Ala Ser Ser Pro Pro Leu
           195                 200                 205
   Trp Leu Ser Arg Ala Pro Pro Pro Ser Pro Pro Ser Gly Pro Glu Thr
       210                 215                 220
   Ala Glu Ala Ala Ala Leu Phe Ser Arg Glu Cys Arg Pro Ser Pro Ala
   225                 230                 235                 240
   Gln Leu Gly Ala Leu Arg Val Met Leu Trp Val Thr Thr Ala Tyr Phe
                   245                 250                 255
   Phe Leu Pro Phe Leu Cys Leu Ser Ile Leu Tyr Gly Leu Ile Gly Arg
               260                 265                 270
   Glu Leu Trp Ser Ser Arg Arg Pro Leu Arg Gly Pro Ala Ala Ser Gly
           275                 280                 285
   Arg Glu Arg Gly His Arg Gln Thr Val Arg Val Leu Leu Val Val Val
       290                 295                 300
   Leu Ala Phe Ile Ile Cys Trp Leu Pro Phe His Val Gly Arg Ile Ile
   305                 310                 315                 320
   Tyr Ile Asn Thr Glu Asp Ser Arg Met Met Tyr Phe Ser Gln Tyr Phe
                   325                 330                 335
   Asn Ile Val Ala Leu Gln Leu Phe Tyr Leu Ser Ala Ser Ile Asn Pro
               340                 345                 350
   Ile Leu Tyr Asn Leu Ile Ser Lys Lys Tyr Arg Ala Ala Ala Phe Lys
           355                 360                 365
   Leu Leu Leu Ala Arg Lys Ser Arg Pro Arg Gly Phe His Arg Ser Arg
       370                 375                 380
   Asp Thr Ala Gly Glu Val Ala Gly Asp Thr Gly Gly Asp Thr Val Gly
   385                 390                 395                 400
   Tyr Thr Glu Thr Ser Ala Asn Val Lys Thr Met Gly
                   405                 410
(2) INFORMATION FOR SEQ ID NO:8:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:8:
      CCATCCTAAT ACGACTCACT ATAGGGC 27
(2) INFORMATION FOR SEQ ID NO:9:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:9:
      TTATCCCATC GTCTTCACGT TAGCGCTTGT CTC 33
(2) INFORMATION FOR SEQ ID NO:10.
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:10:
      CTGCCCTTTC TGTGCCTCAG CATCCTCTAC 30
(2) INFORMATION FOR SEQ ID NO:11:
   (i) SEQUENCE CHARACTERISTICS:
      (A) LENGTH: 900 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:11:
   ATGGGCAGCC CCTGGAACGG CAGCGACGGC CCCGAGGGGG CGCGGGAGCC GCCGTGGCCC   60
   GCGCTGCCGC CTTGCGACGA GCGCCGCTGC TCGCCCTTTC CCCTGGGGGC GCTGGTGCCG  120
   GTGACCGCTG TGTGCCTGTG CCTGTTCGTC GTCGGGGTGA GCGGCAACGT GGTGACCGTG  180
   ATGCTGATCG GGCGCTACCG GGACATGCGG ACCACCACCA ACTTGTACCT GGGCAGCATG  240
   GCCGTGTCCG ACCTACTCAT CCTGCTCGGG CTGCCGTTCG ACCTGTACCG CCTCTGGCGC  300
   TCGCGGCCCT GGGTGTTCGG GCCGCTGCTC TGCCGCCTGT CCCTCTACGT GGGCGAGGGC  360
   TGCACCTACG CCACGCTGCT GCACATGACC GCGCTCAGCG TCGAGCGCTA CCTGGCCATC  420
   TGCCGCCCGC TCCGCGCCCG CGTCTTGGTC ACCCGGCGCC GCGTCCGCGC GCTCATCGCT  480
   GTGCTCTGGG CCGTGGCGCT GCTCTCTGCC GGTCCCTTCT TGTTCCTGGT GGGCGTCGAG  540
   CAGGACCCCG GCATCTCCGT AGTCCCGGGC CTCAATGGCA CCGCGCGGAT CGCCTCCTCG  600
   CCTCTCGCCT CGTCGCCGCC TCTCTGGCTC TCGCGGGCGC CACCGCCGTC CCCGCCGTCG  660
   GGGCCCGAGA CCGCGGAGGC CGCGGCGCTG TTCAGCCGCG AATGCCGGCC GAGCCCCGCG  720
   CAGCTGGGCG CGCTGCGTGT CATGCTGTGG GTCACCACCG CCTACTTCTT CCTGCCCTTT  780
   CTGTGCCTCA GCATCCTCTA CGGGCTCATC GGGCGGGAGC TGTGGAGCAG CCGGCGGCCG  840
   CTGCGAGGCC CGGCCGCCTC GGGGCGGGAG AGAGGCCACC GGCAGACCGT CCGCGTCCTG  900
(2) INFORMATION FOR SEQ ID NO:12:
   (i) SEQUENCE CHARACTERISTICS:
      (A) LENGTH: 300 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:12:
   Met Gly Ser Pro Trp Asn Gly Ser Asp Gly Pro Glu Gly Ala Arg Glu
    1               5                  10                  15
   Pro Pro Trp Pro Ala Leu Pro Pro Cys Asp Glu Arg Arg Cys Ser Pro
               20                  25                  30
   Phe Pro Leu Gly Ala Leu Val Pro Val Thr Ala Val Cys Leu Cys Leu
           35                  40                  45
   Phe Val Val Gly Val Ser Gly Asn Val Val Thr Val Met Leu Ile Gly
       50                  55                  60
   Arg Tyr Arg Asp Met Arg Thr Thr Thr Asn Leu Tyr Leu Gly Ser Met
   65                  70                  75                  80
   Ala Val Ser Asp Leu Leu Ile Leu Leu Gly Leu Pro Phe Asp Leu Tyr
                   85                  90                  95
   Arg Leu Trp Arg Ser Arg Pro Trp Val Phe Gly Pro Leu Leu Cys Arg
               100                 105                 110
   Leu Ser Leu Tyr Val Gly Glu Gly Cys Thr Tyr Ala Thr Leu Leu His
           115                 120                 125
   Met Thr Ala Leu Ser Val Glu Arg Tyr Leu Ala Ile Cys Arg Pro Leu
       130                 135                 140
   Arg Ala Arg Val Leu Val Thr Arg Arg Arg Val Arg Ala Leu Ile Ala
   145                 150                 155                 160
   Val Leu Trp Ala Val Ala Leu Leu Ser Ala Gly Pro Phe Leu Phe Leu
                   165                 170                 175
   Val Gly Val Glu Gln Asp Pro Gly Ile Ser Val Val Pro Gly Leu Asn
               180                 185                 190
   Gly Thr Ala Arg Ile Ala Ser Ser Pro Leu Ala Ser Ser Pro Pro Leu
           195                 200                 205
   Trp Leu Ser Arg Ala Pro Pro Pro Ser Pro Pro Ser Gly Pro Glu Thr
       210                 215                 220
   Ala Glu Ala Ala Ala Leu Phe Ser Arg Glu Cys Arg Pro Ser Pro Ala
   225                 230                 235                 240
   Gln Leu Gly Ala Leu Arg Val Met Leu Trp Val Thr Thr Ala Tyr Phe
                   245                 250                 255
   Phe Leu Pro Phe Leu Cys Leu Ser Ile Leu Tyr Gly Leu Ile Gly Arg
               260                 265                 270
   Glu Leu Trp Ser Ser Arg Arg Pro Leu Arg Gly Pro Ala Ala Ser Gly
           275                 280                 285
   Arg Glu Arg Gly His Arg Gln Thr Val Arg Val Leu
       290                 295                 300
(2) INFORMATION FOR SEQ ID NO:13:
   (i) SEQUENCE CHARACTERISTICS:
      (A) LENGTH: 154 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:13:
   CGTAAGTGGA GCCGCCGTGG TTCCAAAGAC GCCTGCCTGC AGTCCGCCCC GCCGGGGACC    60
   GCGCAAACGC TGGGTCCCCT TCCCCTGCTC GCCCAGCTCT GGGCGCCGCT TCCAGCTCCC   120
   TTTCCTATTT CGATTCCAGC CTCCACCCGC CGGT                               154
(2) INFORMATION FOR SEQ ID NO:14:
   (i) SEQUENCE CHARACTERISTICS:
      (A) LENGTH: 602 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:14:
   AGCTGGTGGT GGTTCTGGCA TTTATAATTT GCTGGTTGCC CTTCCACGTT GGCAGAATCA    60
   TTTACATAAA CACGGAAGAT TCGCGGATGA TGTACTTCTC TCAGTACTTT AACATCGTCG   120
   CTCTGCAACT TTTCTATCTG AGCGCATCTA TCAACCCAAT CCTCTACAAC CTCATTTCAA   180
   AGAAGTACAG AGCGGCGGCC TTTAAACTGC TGCTCGCAAG GAAGTCCAGG CCGAGAGGCT   240
   TCCACAGAAG CAGGGACACT GCGGGGGAAG TTGCAGGGGA CACTGGAGGA GACACGGTGG   300
   GCTACACCGA GACAAGCGCT AACGTGAAGA CGATGGGATA ACGTAAGTGG AGCCGCCGTG   360
   GTTCCAAAGA CGCCTGCCTG CAGTCCGCCC CGCCGGGGAC CGCGCAAACG CTGGGTCCCC   420
   TTCCCCTGCT CGCCCAGCTC TGGGCGCCGC TTCCAGCTCC CTTTCCTATT TCGATTCCAG   480
   CCTCCACCCG CCGTGGTGGT GGTTCTGGCA TTTATAATTT GCTGGTTGCC CTTCCACGTT   540
   GGCAGAATCA TTTACATAAA CACGGAAGAT TCGCGGATGA TGTACTTCTC TCAGTACTTT   600
   AA                                                                  602
(2) INFORMATION FOR SEQ ID NO:15:
   (i) SEQUENCE CHARACTERISTICS:
      (A) LENGTH: 198 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:15:
   Leu Val Val Val Leu Ala Phe Ile Ile Cys Trp Leu Pro Phe His Val
    1               5                  10                  15
   Gly Arg Ile Ile Tyr Ile Asn Thr Glu Asp Ser Arg Met Met Tyr Phe
               20                  25                  30
   Ser Gln Tyr Phe Asn Ile Val Ala Leu Gln Leu Phe Tyr Leu Ser Ala
           35                  40                  45
   Ser Ile Asn Pro Ile Leu Tyr Asn Leu Ile Ser Lys Lys Tyr Arg Ala
       50                  55                  60
   Ala Ala Phe Lys Leu Leu Leu Ala Arg Lys Ser Arg Pro Arg Gly Phe
   65                  70                  75                  80
   His Arg Ser Arg Asp Thr Ala Gly Glu Val Ala Gly Asp Thr Gly Gly
                   85                  90                  95
   Asp Thr Val Gly Tyr Thr Glu Thr Ser Ala Asn Val Lys Thr Met Gly
               100                 105                 110
   Arg Lys Trp Ser Arg Arg Gly Ser Lys Asp Ala Cys Leu Gln Ser Ala
           115                 120                 125
   Pro Pro Gly Thr Ala Gln Thr Leu Gly Pro Leu Pro Leu Leu Ala Gln
       130                 135                 140
   Leu Trp Ala Pro Leu Pro Ala Pro Phe Pro Ile Ser Ile Pro Ala Ser
   145                 150                 155                 160
   Thr Arg Arg Gly Gly Gly Ser Gly Ile Tyr Asn Leu Leu Val Ala Leu
                   165                 170                 175
   Pro Arg Trp Gln Asn His Leu His Lys His Gly Arg Phe Ala Asp Asp
               180                 185                 190
   Val Leu Leu Ser Val Leu
           195

### SEQUENCE LISTING

<110> Merck & Co., Inc.
<120> CLONING AND IDENTIFICATION OF THE MOTILIN RECEPTOR
<130> 20251 PCT
<140> 60/089,098
   <141> 1998-06-12
<150> PCT/US99/12773
   <151> 1999-06-08
<160> 15
<170> FastSEQ for Windows Version 4.0
<210> 1
   <211> 3066
   <212> DNA
   <213> Homo sapiens
<400> 1
   ttgaaattat ctggtcactg ccgggcgcgg tggctcacgc ctgtaatccc agcactttgg    60
   gaggtcgagg cgggtggacc acctggggtc aggagttcga gaccaggctg gccaacatgg   120
   cgaaaccctg actacacaaa aaacacaaaa tttagccggg gcttgggcgc tcctgtgctc   180
   ccagctactc aggaggctga ggtgggagga ctgcttgagc ctgggaggtc gaggctgcag   240
   tgagctgtga tcgcgccact taaactccag cctggacgac agtgagaccc tgtctcaaga   300
   agaaaaaaag aaagaaagaa agaaaaaaag aaaaaaaaga aattatttgg tcaattatat   360
   ggtcagctcc ctccaccact cgcgaattta cagaagagga gaactgggct gggcgagacc   420
   aggactagcc caagattaca caagttactc ggttgtagag ccaggattag acaggagagg   480
   ctctagattc tggtctagac tcccctccta ttatttagca ttatggcttc ctgaggatta   540
   ccatgagccc tcctccaccg tcaagcggca gctaccagcc accagaccag atcccttcga   600
   aggtgcccgg agtaccagac tgacaaaagc gcccgtacag tgctcagtcc tgtaaccaaa   660
   gctgtctagg gtgcagacat cgctcaccgg accgggtagg gctcgtgcgc taagggcgcc   720
   gggtattcca gttagtggag agggaagcgc cctggaactg catgggcccg ggagagggcg   780
   cgggagcgga gcatggccgg gccggggcgg gccgcggccg tgggcggaga ctgcgcgcag   840
   ctagctcggg agcgcctcgg agcccacccc gcagagccgc ttctcgcgcc ccgcagcgca   900
   gcgcagcgct ccgccgtctg acctgccgcg cccgcagcgt gcgggctggg aaaggaggcg   960
   ctcaccgaga gggaccacgc gccaggctcc cagcccgacc cgggacgcgg cggccgcgcg  1020
   gagcacccat gggcagcccc tggaacggca gcgacggccc cgagggggcg cgggagccgc   1080
   cgtggcccgc gctgccgcct tgcgacgagc gccgctgctc gccctttccc ctgggggcgc  1140
   tggtgccggt gaccgctgtg tgcctgtgcc tgttcgtcgt cggggtgagc ggcaacgtgg  1200
   tgaccgtgat gctgatcggg cgctaccggg acatgcggac caccaccaac ttgtacctgg  1260
   gcagcatggc cgtgtccgac ctactcatcc tgctcgggct gccgttcgac ctgtaccgcc  1320
   tctggcgctc gcggccctgg gtgttcgggc cgctgctctg ccgcctgtcc ctctacgtgg  1380
   gcgagggctg cacctacgcc acgctgctgc acatgaccgc gctcagcgtc gagcgctacc  1440
   tggccatctg ccgcccgctc cgcgcccgcg tcttggtcac ccggcgccgc gtccgcgcgc  1500
   tcatcgctgt gctctgggcc gtggcgctgc tctctgccgg tcccttcttg ttcctggtgg  1560
   gcgtcgagca ggaccccggc atctccgtag tcccgggcct caatggcacc gcgcggatcg  1620
   cctcctcgcc tctcgcctcg tcgccgcctc tctggctctc gcgggcgcca ccgccgtccc  1680
   cgccgtcggg gcccgagacc gcggaggccg cggcgctgtt cagccgcgaa tgccggccga  1740
   gccccgcgca gctgggcgcg ctgcgtgtca tgctgtgggt caccaccgcc tacttcttcc  1800
   tgccctttct gtgcctcagc atcctctacg ggctcatcgg gcgggagctg tggagcagcc  1860
   ggcggccgct gcgaggcccg gccgcctcgg ggcgggagag aggccaccgg cagaccgtcc  1920
   gcgtcctgcg taagtggagc cgccgtggtt ccaaagacgc ctgcctgcag tccgccccgc  1980
   cggggaccgc gcaaacgctg ggtccccttc ccctgctcgc ccagctctgg gcgccgcttc  2040
   cagctccctc ctatttcgat tccagcctcc acccgccggt acttcccatc ccccgagaaa  2100
   accatgtcct gtcccccagg agctctgggg gaccccaggg cgctttgagg gtgggatccc  2160
   cggatccgat tcagtaacca gcagtgcttt tccagagcct ctgagaccag aaaggagagt  2220
   tggtaattct taatccaacc acctgttaga tgccacaaat gaggagtcct cacagtgctc  2280
   ttgagaagac gagggagatt tcattaagct aaaatttttt atttaatgtt aagtgatgct  2340
   gaaggctaaa gtaaaccttg ctcgtatcaa aaagtaaaga ttgtgcagac ctgttgtaga  2400
   attcttttca acagagaaca gaaaacttgt ctccgaagtg ggtttgtgga aggaagcctg  2460
   ccaaggcggc ttgttcagag aaattgctcc ttctggttta tgtccagcct tgataacaca  2520
   tatgggagcc tactatgcag ttttaaagca agtatccatg cagcctgcag cctggtcatt  2580
   ttttctgggg tgaggatctg cctaggtaga agttttctct aatttatttt gctgttactt  2640
   gttattgcag atggttcctt gtcggggtgg ggggtttatt tgcttcccaa tgcttttgtt  2700
   aatcccggtg ctgtgtctta tgttgcagtg gtggtggttc tggcatttat aatttgctgg  2760
   ttgcccttcc acgttggcag aatcatttac ataaacacgg aagattcgcg gatgatgtac  2820
   ttctctcagt actttaacat cgtcgctctg caacttttct atctgagcgc atctatcaac  2880
   ccaatcctct acaacctcat ttcaaagaag tacagagcgg cggcctttaa actgctgctc  2940
   gcaaggaagt ccaggccgag aggcttccac agaagcaggg acactgcggg ggaagttgca  3000
   ggggacactg gaggagacac ggtgggctac accgagacaa gcgctaacgt gaagacgatg  3060
   ggataa                                                             3066
<210> 2
   <211> 1239
   <212> DNA
   <213> Homo sapiens
<400> 2
   atgggcagcc cctggaacgg cagcgacggc cccgaggggg cgcgggagcc gccgtggccc     60
   gcgctgccgc cttgcgacga gcgccgctgc tcgccctttc ccctgggggc gctggtgccg     120
   gtgaccgctg tgtgcctgtg cctgttcgtc gtcggggtga gcggcaacgt ggtgaccgtg    180
   atgctgatcg ggcgctaccg ggacatgcgg accaccacca acttgtacct gggcagcatg    240
   gccgtgtccg acctactcat cctgctcggg ctgccgttcg acctgtaccg cctctggcgc    300
   tcgcggccct gggtgttcgg gccgctgctc tgccgcctgt ccctctacgt gggcgagggc    360
   tgcacctacg ccacgctgct gcacatgacc gcgctcagcg tcgagcgcta cctggccatc    420
   tgccgcccgc tccgcgcccg cgtcttggtc acccggcgcc gcgtccgcgc gctcatcgct    480
   gtgctctggg ccgtggcgct gctctctgcc ggtcccttct tgttcctggt gggcgtcgag    540
   caggaccccg gcatctccgt agtcccgggc ctcaatggca ccgcgcggat cgcctcctcg    600
   cctctcgcct cgtcgccgcc tctctggctc tcgcgggcgc caccgccgtc cccgccgtcg    660
   gggcccgaga ccgcggaggc cgcggcgctg ttcagccgcg aatgccggcc gagccccgcg    720
   cagctgggcg cgctgcgtgt catgctgtgg gtcaccaccg cctacttctt cctgcccttt    780
   ctgtgcctca gcatcctcta cgggctcatc gggcgggagc tgtggagcag ccggcggccg    840
   ctgcgaggcc cggccgcctc ggggcgggag agaggccacc ggcagaccgt ccgcgtcctg    900
   ctggtggtgg ttctggcatt tataatttgc tggttgccct tccacgttgg cagaatcatt    960
   tacataaaca cggaagattc gcggatgatg tacttctctc agtactttaa catcgtcgct   1020
   ctgcaacttt tctatctgag cgcatctatc aacccaatcc tctacaacct catttcaaag   1080
   aagtacagag cggcggcctt taaactgctg ctcgcaagga agtccaggcc gagaggcttc   1140
   cacagaagca gggacactgc gggggaagtt gcaggggaca ctggaggaga cacggtgggc   1200
   tacaccgaga caagcgctaa cgtgaagacg atgggataa                           1239
<210> 3
   <211> 412
   <212> PRT
   <213> Homo sapiens
<400> 3
   Met Gly Ser Pro Trp Asn Gly Ser Asp Gly Pro Glu Gly Ala Arg Glu
    1               5                  10                  15
   Pro Pro Trp Pro Ala Leu Pro Pro Cys Asp Glu Arg Arg Cys Ser Pro
               20                  25                  30
   Phe Pro Leu Gly Ala Leu Val Pro Val Thr Ala Val Cys Leu Cys Leu
           35                  40                  45
   Phe Val Val Gly Val Ser Gly Asn Val Val Thr Val Met Leu Ile Gly
       50                  55                  60
   Arg Tyr Arg Asp Met Arg Thr Thr Thr Asn Leu Tyr Leu Gly Ser Met
   65                  70                  75                  80
   Ala Val Ser Asp Leu Leu Ile Leu Leu Gly Leu Pro Phe Asp Leu Tyr
                   85                  90                  95
   Arg Leu Trp Arg Ser Arg Pro Trp Val Phe Gly Pro Leu Leu Cys Arg
               100                 105                 110
   Leu Ser Leu Tyr Val Gly Glu Gly Cys Thr Tyr Ala Thr Leu Leu His
           115                 120                 125
   Met Thr Ala Leu Ser Val Glu Arg Tyr Leu Ala Ile Cys Arg Pro Leu
       130                 135                 140
   Arg Ala Arg Val Leu Val Thr Arg Arg Arg Val Arg Ala Leu Ile Ala
   145                 150                 155                 160
   Val Leu Trp Ala Val Ala Leu Leu Ser Ala Gly Pro Phe Leu Phe Leu
                   165                 170                 175
   Val Gly Val Glu Gln Asp Pro Gly Ile Ser Val Val Pro Gly Leu Asn
               180                 185                 190
   Gly Thr Ala Arg Ile Ala Ser Ser Pro Leu Ala Ser Ser Pro Pro Leu
           195                 200                 205
   Trp Leu Ser Arg Ala Pro Pro Pro Ser Pro Pro Ser Gly Pro Glu Thr
       210                 215                 220
   Ala Glu Ala Ala Ala Leu Phe Ser Arg Glu Cys Arg Pro Ser Pro Ala
   225                 230                 235                 240
   Gln Leu Gly Ala Leu Arg Val Met Leu Trp Val Thr Thr Ala Tyr Phe
                   245                 250                 255
   Phe Leu Pro Phe Leu Cys Leu Ser Ile Leu Tyr Gly Leu Ile Gly Arg
               260                 265                 270
   Glu Leu Trp Ser Ser Arg Arg Pro Leu Arg Gly Pro Ala Ala Ser Gly
           275                 280                 285
   Arg Glu Arg Gly His Arg Gln Thr Val Arg Val Leu Leu Val Val Val
       290                 295                 300
   Leu Ala Phe Ile Ile Cys Trp Leu Pro Phe His Val Gly Arg Ile Ile
   305                 310                 315                 320
   Tyr Ile Asn Thr Glu Asp Ser Arg Met Met Tyr Phe Ser Gln Tyr Phe
                   325                 330                 335
   Asn Ile Val Ala Leu Gln Leu Phe Tyr Leu Ser Ala Ser Ile Asn Pro
               340                 345                 350
   Ile Leu Tyr Asn Leu Ile Ser Lys Lys Tyr Arg Ala Ala Ala Phe Lys
           355                 360                 365
   Leu Leu Leu Ala Arg Lys Ser Arg Pro Arg Gly Phe His Arg Ser Arg
       370                 375                 380
   Asp Thr Ala Gly Glu Val Ala Gly Asp Thr Gly Gly Asp Thr Val Gly
   385                 390                 395                 400
   Tyr Thr Glu Thr Ser Ala Asn Val Lys Thr Met Gly
                   405                 410
<210> 4
   <211> 1390
   <212> DNA
   <213> Homo sapiens
<400> 4
   atgggcagcc cctggaacgg cagcgacggc cccgaggggg cgcgggagcc gccgtggccc      60
   gcgctgccgc cttgcgacga gcgccgctgc tcgccctttc ccctgggggc gctggtgccg     120
   gtgaccgctg tgtgcctgtg cctgttcgtc gtcggggtga gcggcaacgt ggtgaccgtg     180
   atgctgatcg ggcgctaccg ggacatgcgg accaccacca acttgtacct gggcagcatg     240
   gccgtgtccg acctactcat cctgctcggg ctgccgttcg acctgtaccg cctctggcgc     300
   tcgcggccct gggtgttcgg gccgctgctc tgccgcctgt ccctctacgt gggcgagggc     360
   tgcacctacg ccacgctgct gcacatgacc gcgctcagcg tcgagcgcta cctggccatc     420
   tgccgcccgc tccgcgcccg cgtcttggtc acccggcgcc gcgtccgcgc gctcatcgct     480
   gtgctctggg ccgtggcgct gctctctgcc ggtcccttct tgttcctggt gggcgtcgag     540
   caggaccccg gcatctccgt agtcccgggc ctcaatggca ccgcgcggat cgcctcctcg     600
   cctctcgcct cgtcgccgcc tctctggctc tcgcgggcgc caccgccgtc cccgccgtcg     660
   gggcccgaga ccgcggaggc cgcggcgctg ttcagccgcg aatgccggcc gagccccgcg     720
   cagctgggcg cgctgcgtgt catgctgtgg gtcaccaccg cctacttctt cctgcccttt     780
   ctgtgcctca gcatcctcta cgggctcatc gggcgggagc tgtggagcag ccggcggccg     840
   ctgcgaggcc cggccgcctc ggggcgggag agaggccacc ggcagaccgt ccgcgtcctg     900
   cgtaagtgga gccgccgtgg ttccaaagac gcctgcctgc agtccgcccc gccggggacc     960
   gcgcaaacgc tgggtcccct tcccctgctc gcccagctct gggcgccgct tccagctccc    1020
   tttcctattt cgattccagc ctccacccgc cgtggtggtg gttctggcat ttataatttg    1080
   ctggttgccc ttccacgttg gcagaatcat ttacataaac acggaagatt cgcggatgat    1140
   gtacttctct cagtacttta acatcgtcgc tctgcaactt ttctatctga gcgcatctat    1200
   caacccaatc ctctacaacc tcatttcaaa gaagtacaga gcggcggcct ttaaactgct    1260
   gctcgcaagg aagtccaggc cgagaggctt ccacagaagc agggacactg cgggggaagt    1320
   tgcaggggac actggaggag acacggtggg ctacaccgag acaagcgcta acgtgaagac    1380
   gatgggataa                                                           1390
<210> 5
   <211> 386
   <212> PRT
   <213> Homo sapiens
<400> 5
   Met Gly Ser Pro Trp Asn Gly Ser Asp Gly Pro Glu Gly Ala Arg Glu
    1               5                  10                  15
   Pro Pro Trp Pro Ala Leu Pro Pro Cys Asp Glu Arg Arg Cys Ser Pro
               20                  25                  30
   Phe Pro Leu Gly Ala Leu Val Pro Val Thr Ala Val Cys Leu Cys Leu
           35                  40                  45
   Phe Val Val Gly Val Ser Gly Asn Val Val Thr Val Met Leu Ile Gly
       50                  55                  60
   Arg Tyr Arg Asp Met Arg Thr Thr Thr Asn Leu Tyr Leu Gly Ser Met
   65                  70                  75                  80
   Ala Val Ser Asp Leu Leu Ile Leu Leu Gly Leu Pro Phe Asp Leu Tyr
                   85                  90                  95
   Arg Leu Trp Arg Ser Arg Pro Trp Val Phe Gly Pro Leu Leu Cys Arg
               100                 105                 110
   Leu Ser Leu Tyr Val Gly Glu Gly Cys Thr Tyr Ala Thr Leu Leu His
           115                 120                 125
   Met Thr Ala Leu Ser Val Glu Arg Tyr Leu Ala Ile Cys Arg Pro Leu
       130                 135                 140
   Arg Ala Arg Val Leu Val Thr Arg Arg Arg Val Arg Ala Leu Ile Ala
   145                 150                 155                 160
   Val Leu Trp Ala Val Ala Leu Leu Ser Ala Gly Pro Phe Leu Phe Leu
                   165                 170                 175
   Val Gly Val Glu Gln Asp Pro Gly Ile Ser Val Val Pro Gly Leu Asn
               180                 185                 190
   Gly Thr Ala Arg Ile Ala Ser Ser Pro Leu Ala Ser Ser Pro Pro Leu
           195                 200                 205
   Trp Leu Ser Arg Ala Pro Pro Pro Ser Pro Pro Ser Gly Pro Glu Thr
       210                 215                 220
   Ala Glu Ala Ala Ala Leu Phe Ser Arg Glu Cys Arg Pro Ser Pro Ala
   225                 230                 235                 240
   Gln Leu Gly Ala Leu Arg Val Met Leu Trp Val Thr Thr Ala Tyr Phe
                   245                 250                 255
   Phe Leu Pro Phe Leu Cys Leu Ser Ile Leu Tyr Gly Leu Ile Gly Arg
               260                 265                 270
   Glu Leu Trp Ser Ser Arg Arg Pro Leu Arg Gly Pro Ala Ala Ser Gly
           275                 280                 285
   Arg Glu Arg Gly His Arg Gln Thr Val Arg Val Leu Arg Lys Trp Ser
       290                 295                 300
   Arg Arg Gly Ser Lys Asp Ala Cys Leu Gln Ser Ala Pro Pro Gly Thr
   305                 310                 315                 320
   Ala Gln Thr Leu Gly Pro Leu Pro Leu Leu Ala Gln Leu Trp Ala Pro
                   325                 330                 335
   Leu Pro Ala Pro Phe Pro Ile Ser Ile Pro Ala Ser Thr Arg Arg Gly
               340                 345                 350
   Gly Gly Ser Gly Ile Tyr Asn Leu Leu Val Ala Leu Pro Arg Trp Gln
           355                 360                 365
   Asn His Leu His Lys His Gly Arg Phe Ala Asp Asp Val Leu Leu Ser
       370                 375                 380
   Val Leu
   385
<210> 6
   <211> 1092
   <212> DNA
   <213> Spheroides nephelus
<400> 6
   atgccctgga ccagacccca ggtggacctc catgctgctg cagcagagac catggaccag      60
   tacaccacgg acgaccacca ctacgagggc tccctcttcc ccgcgtccac cctcatcccc     120
   gtcacggtca tctgcatcct catcttcgtg gtcggcgtga ccggcaacac catgaccatc     180
   ctcatcatcc agtacttcaa ggacatgaag accaccacca acctgtacct gtccagcatg     240
   gccgtgtccg acctcgtcat cttcctctgc ctgcccttcg acctgtaccg cctgtggaag     300
   tacgtgccgt ggctgttcgg cgaggccgtg tgccgcctct accactacat cttcgaaggc     360
   tgcacgtcgg ccaccatcct ccacatcacg gccctgagca tcgagcgcta cctggccatc     420
   agcttccccc tcaggagcaa ggtgatggtg accaggagaa gggtccagta catcatcctg     480
   gccctgtggt gcttcgccct ggtgtcggcc gctcccacgc tcttcctggt cggggtggag     540
   tacgacaacg agacgcaccc cgactacaac acgggccagt gcaagcacac gggctacgcc     600
   atcagctcgg ggcagctgca catcatgatc tgggtgtcca ccacctactt cttctgcccg     660
   atgctgtgtc tcctcttcct ctacggctcc atcgggtgca agctgtggaa gagcaagaac     720
   gacctgcagg gcccgtgcgc cctggcccgc gagaggtcgc acaggcaaac ggtgaagatc     780
   ctggtggtgg tggtgctggc cttcatcatc tgctggctgc cctaccacat cggcaggaac     840
   ctgttcgccc aggtggacga ctacgacacg gccatgctca gccagaattt caacatggcc     900
   tccatggtgc tctgctacct cagcgcctcc atcaaccccg tcgtctacaa cctgatgtcg     960
   aggaagtacc gggccgccgc caagcgcctc ttcctgctcc accagagacc caagccggcc    1020
   caccgggggc aggggcagtt ttgcatgatc ggccacagcc ccaccctgga cgagagcctg    1080
   acgggggtgt ga                                                        1092
<210> 7
   <211> 363
   <212> PRT
   <213> Spheroides nephelus
<400> 7
   Met Pro Trp Thr Arg Pro Gln Val Asp Leu His Ala Ala Ala Ala Glu
   1               5                   10                  15
   Thr Met Asp Gln Tyr Thr Thr Asp Asp His His Tyr Glu Gly Ser Leu
               20                  25                  30
   Phe Pro Ala Ser Thr Leu Ile Pro Val Thr Val Ile Cys Ile Leu Ile
           35                  40                  45
   Phe Val Val Gly Val Thr Gly Asn Thr Met Thr Ile Leu Ile Ile Gln
       50                  55                  60
   Tyr Phe Lys Asp Met Lys Thr Thr Thr Asn Leu Tyr Leu Ser Ser Met
   65                  70                  75                  80
   Ala Val Ser Asp Leu Val Ile Phe Leu Cys Leu Pro Phe Asp Leu Tyr
                   85                  90                  95
   Arg Leu Trp Lys Tyr Val Pro Trp Leu Phe Gly Glu Ala Val Cys Arg
               100                 105                 110
   Leu Tyr His Tyr Ile Phe Glu Gly Cys Thr Ser Ala Thr Ile Leu His
           115                 120                 125
   Ile Thr Ala Leu Ser Ile Glu Arg Tyr Leu Ala Ile Ser Phe Pro Leu
       130                 135                 140
   Arg Ser Lys Val Met Val Thr Arg Arg Arg Val Gln Tyr Ile Ile Leu
   145                 150                 155                 160
   Ala Leu Trp Cys Phe Ala Leu Val Ser Ala Ala Pro Thr Leu Phe Leu
                   165                 170                 175
   Val Gly Val Glu Tyr Asp Asn Glu Thr His Pro Asp Tyr Asn Thr Gly
               180                 185                 190
   Gln Cys Lys His Thr Gly Tyr Ala Ile Ser Ser Gly Gln Leu His Ile
           195                 200                 205
   Met Ile Trp Val Ser Thr Thr Tyr Phe Phe Cys Pro Met Leu Cys Leu
       210                 215                 220
   Leu Phe Leu Tyr Gly Ser Ile Gly Cys Lys Leu Trp Lys Ser Lys Asn
   225                 230                 235                 240
   Asp Leu Gln Gly Pro Cys Ala Leu Ala Arg Glu Arg Ser His Arg Gln
                   245                 250                 255
   Thr Val Lys Ile Leu Val Val Val Val Leu Ala Phe Ile Ile Cys Trp
               260                 265                 270
   Leu Pro Tyr His Ile Gly Arg Asn Leu Phe Ala Gln Val Asp Asp Tyr
           275                 280                 285
   Asp Thr Ala Met Leu Ser Gln Asn Phe Asn Met Ala Ser Met Val Leu
       290                 295                 300
   Cys Tyr Leu Ser Ala Ser Ile Asn Pro Val Val Tyr Asn Leu Met Ser
   305                 310                 315                 320
   Arg Lys Tyr Arg Ala Ala Ala Lys Arg Leu Phe Leu Leu His Gln Arg
                   325                 330                 335
   Pro Lys Pro Ala His Arg Gly Gln Gly Gln Phe Cys Met Ile Gly His
               340                 345                 350
   Ser Pro Thr Leu Asp Glu Ser Leu Thr Gly Val
           355                     360
<210> 8
   <211> 27
   <212> DNA
   <213> Artificial Sequence
<220>
   <223> PCR Primer
<400> 8
   ccatcctaat acgactcact atagggc 27
<210> 9
   <211> 33
   <212> DNA
   <213> Artificial Sequence
<220>
   <223> PCR Primer
<400> 9
   ttatcccatc gtcttcacgt tagcgcttgt ctc 33
<210> 10
   <211> 30
   <212> DNA
   <213> Artificial Sequence
<220>
   <223> PCR Primer
<400> 10
   ctgccctttc tgtgcctcag catcctctac 30
<210> 11
   <211> 900
   <212> DNA
   <213> Homo sapiens
<400> 11
   atgggcagcc cctggaacgg cagcgacggc cccgaggggg cgcgggagcc gccgtggccc     60
   gcgctgccgc cttgcgacga gcgccgctgc tcgccctttc ccctgggggc gctggtgccg    120
   gtgaccgctg tgtgcctgtg cctgttcgtc gtcggggtga gcggcaacgt ggtgaccgtg    180
   atgctgatcg ggcgctaccg ggacatgcgg accaccacca acttgtacct gggcagcatg    240
   gccgtgtccg acctactcat cctgctcggg ctgccgttcg acctgtaccg cctctggcgc    300
   tcgcggccct gggtgttcgg gccgctgctc tgccgcctgt ccctctacgt gggcgagggc    360
   tgcacctacg ccacgctgct gcacatgacc gcgctcagcg tcgagcgcta cctggccatc    420
   tgccgcccgc tccgcgcccg cgtcttggtc acccggcgcc gcgtccgcgc gctcatcgct    480
   gtgctctggg ccgtggcgct gctctctgcc ggtcccttct tgttcctggt gggcgtcgag    540
   caggaccccg gcatctccgt agtcccgggc ctcaatggca ccgcgcggat cgcctcctcg    600
   cctctcgcct cgtcgccgcc tctctggctc tcgcgggcgc caccgccgtc cccgccgtcg    660
   gggcccgaga ccgcggaggc cgcggcgctg ttcagccgcg aatgccggcc gagccccgcg    720
   cagctgggcg cgctgcgtgt catgctgtgg gtcaccaccg cctacttctt cctgcccttt    780
   ctgtgcctca gcatcctcta cgggctcatc gggcgggagc tgtggagcag ccggcggccg     840
   ctgcgaggcc cggccgcctc ggggcgggag agaggccacc ggcagaccgt ccgcgtcctg     900
<210> 12
   <211> 300
   <212> PRT
   <213> Homo sapiens
<400> 12
   Met Gly Ser Pro Trp Asn Gly Ser Asp Gly Pro Glu Gly Ala Arg Glu
    1               5                  10                  15
   Pro Pro Trp Pro Ala Leu Pro Pro Cys Asp Glu Arg Arg Cys Ser Pro
               20                  25                  30
   Phe Pro Leu Gly Ala Leu Val Pro Val Thr Ala Val Cys Leu Cys Leu
           35                  40                  45
   Phe Val Val Gly Val Ser Gly Asn Val Val Thr Val Met Leu Ile Gly
       50                  55                  60
   Arg Tyr Arg Asp Met Arg Thr Thr Thr Asn Leu Tyr Leu Gly Ser Met
   65                  70                  75                  80
   Ala Val Ser Asp Leu Leu Ile Leu Leu Gly Leu Pro Phe Asp Leu Tyr
                   85                  90                  95
   Arg Leu Trp Arg Ser Arg Pro Trp Val Phe Gly Pro Leu Leu Cys Arg
               100                 105                 110
   Leu Ser Leu Tyr Val Gly Glu Gly Cys Thr Tyr Ala Thr Leu Leu His
           115                 120                 125
   Met Thr Ala Leu Ser Val Glu Arg Tyr Leu Ala Ile Cys Arg Pro Leu
       130                 135                 140
   Arg Ala Arg Val Leu Val Thr Arg Arg Arg Val Arg Ala Leu Ile Ala
   145                 150                 155                 160
   Val Leu Trp Ala Val Ala Leu Leu Ser Ala Gly Pro Phe Leu Phe Leu
                   165                 170                 175
   Val Gly Val Glu Gln Asp Pro Gly Ile Ser Val Val Pro Gly Leu Asn
               180                 185                 190
   Gly Thr Ala Arg Ile Ala Ser Ser Pro Leu Ala Ser Ser Pro Pro Leu
           195                 200                 205
   Trp Leu Ser Arg Ala Pro Pro Pro Ser Pro Pro Ser Gly Pro Glu Thr
       210                 215                 220
   Ala Glu Ala Ala Ala Leu Phe Ser Arg Glu Cys Arg Pro Ser Pro Ala
   225                 230                 235                 240
   Gln Leu Gly Ala Leu Arg Val Met Leu Trp Val Thr Thr Ala Tyr Phe
                   245                 250                 255
   Phe Leu Pro Phe Leu Cys Leu Ser Ile Leu Tyr Gly Leu Ile Gly Arg
               260                 265                 270
   Glu Leu Trp Ser Ser Arg Arg Pro Leu Arg Gly Pro Ala Ala Ser Gly
           275                 280                 285
   Arg Glu Arg Gly His Arg Gln Thr Val Arg Val Leu
       290                 295                 300
<210> 13
   <211> 154
   <212> DNA
   <213> Homo sapiens
<400> 13
   cgtaagtgga gccgccgtgg ttccaaagac gcctgcctgc agtccgcccc gccggggacc      60
   gcgcaaacgc tgggtcccct tcccctgctc gcccagctct gggcgccgct tccagctccc     120
   tttcctattt cgattccagc ctccacccgc cggt                                 154
<210> 14
   <211> 602
   <212> DNA
   <213> Homo sapiens
<400> 14
   agctggtggt ggttctggca tttataattt gctggttgcc cttccacgtt ggcagaatca      60
   tttacataaa cacggaagat tcgcggatga tgtacttctc tcagtacttt aacatcgtcg     120
   ctctgcaact tttctatctg agcgcatcta tcaacccaat cctctacaac ctcatttcaa     180
   agaagtacag agcggcggcc tttaaactgc tgctcgcaag gaagtccagg ccgagaggct     240
   tccacagaag cagggacact gcgggggaag ttgcagggga cactggagga gacacggtgg     300
   gctacaccga gacaagcgct aacgtgaaga cgatgggata acgtaagtgg agccgccgtg     360
   gttccaaaga cgcctgcctg cagtccgccc cgccggggac cgcgcaaacg ctgggtcccc     420
   ttcccctgct cgcccagctc tgggcgccgc ttccagctcc ctttcctatt tcgattccag     480
   cctccacccg ccgtggtggt ggttctggca tttataattt gctggttgcc cttccacgtt     540
   ggcagaatca tttacataaa cacggaagat tcgcggatga tgtacttctc tcagtacttt     600
   aa                                                                    602
<210> 15
   <211> 198
   <212> PRT
   <213> Homo sapiens
<400> 15
   Leu Val Val Val Leu Ala Phe Ile Ile Cys Trp Leu Pro Phe His Val
    1               5                  10                  15
   Gly Arg Ile Ile Tyr Ile Asn Thr Glu Asp Ser Arg Met Met Tyr Phe
               20                  25                  30
   Ser Gln Tyr Phe Asn Ile Val Ala Leu Gln Leu Phe Tyr Leu Ser Ala
           35                  40                  45
   Ser Ile Asn Pro Ile Leu Tyr Asn Leu Ile Ser Lys Lys Tyr Arg Ala
       50                  55                  60
   Ala Ala Phe Lys Leu Leu Leu Ala Arg Lys Ser Arg Pro Arg Gly Phe
   65                  70                  75                  80
   His Arg Ser Arg Asp Thr Ala Gly Glu Val Ala Gly Asp Thr Gly Gly
                   85                  90                  95
   Asp Thr Val Gly Tyr Thr Glu Thr Ser Ala Asn Val Lys Thr Met Gly
               100                 105                 110
   Arg Lys Trp Ser Arg Arg Gly Ser Lys Asp Ala Cys Leu Gln Ser Ala
           115                 120                 125
   Pro Pro Gly Thr Ala Gln Thr Leu Gly Pro Leu Pro Leu Leu Ala Gln
       130                 135                 140
   Leu Trp Ala Pro Leu Pro Ala Pro Phe Pro Ile Ser Ile Pro Ala Ser
   145                 150                 155                 160
   Thr Arg Arg Gly Gly Gly Ser Gly Ile Tyr Asn Leu Leu Val Ala Leu
                   165                 170                 175
   Pro Arg Trp Gln Asn His Leu His Lys His Gly Arg Phe Ala Asp Asp
               180                 185                 190
   Val Leu Leu Ser Val Leu
           195